# EUROPÄISCHE PATENTSCHRIFT

(11) **EP 1 663 141 B1**
(45) Veröffentlichungstag und Bekanntmachung des Hinweises auf die Patenterteilung: **21.03.2007**
(21) Anmeldenummer: 04740995.8
(22) Anmeldetag: 14.07.2004
(51) Int. Cl.: A61K 9/00, A61K 31/4174

(54) **WÄSSRIGE PHARMAZEUTISCHE LÖSUNG ENTHALTEND OXYMETAZOLIN UND/ODER XYLOMETAZOLIN**
AQUEOUS PHARMACEUTICAL SOLUTION CONTAINING OXYMETAZOLINE AND/OR XYLOMETAZOLINE
SOLUTION PHARMACEUTIQUE AQUEUSE CONTENANT DE L'OXYMETAZOLINE ET/OU DE LA XYLOMETAZOLINE

(30) Priorität: 13.08.2003 DE 10337186
(43) Veröffentlichungstag der Anmeldung: 07.06.2006
(73) Patentinhaber: Merck Patent GmbH, 64293 Darmstadt (DE)
(72) Erfinder: BÖHME, Frank, 64342 Seeheim-Jugenheim (DE); ESCHENBACH, Bernd, 65795 Hattersheim (DE); FÄRBER, Dagmar, 64646 Heppenheim (DE); HEY, Claudia, 64283 Darmstadt (DE); PFAFF, Barbara, 63825 Schöllkrippen (DE); TSCHAIKIN, Marion, 64297 Darmstadt (DE)
(86) Internationale Anmeldenummer: PCT/EP2004/007780
(87) Internationale Veröffentlichungsnummer: WO 2005/018601

(56) Entgegenhaltungen:
- WO-A-00/78297
- WO-A-02/24116

## Beschreibung

Die vorliegende Erfindung betrifft eine stabile wässrige Lösung enthaltend Oxymetazolin und/oder Xylometazolin, ein Zinksalz und ein Puffersalz. Die wässrige Lösung ist insbesondere zur lokalen Verabreichung in die Nase zur Schleimhautabschwellung geeignet.

Oxymetazolin [6-tert-Butyl-3-(4,5-dihydro-1-*H*-imidzol-2- ylmethyl)-2,4-dimethylphenol] und Xylometazolin [2- (4-tert.Butyl-2,6-dimethylbenzyl)-4,5-dihydro-1-*H*-imidazol] sind vasokonstriktorisch wirkende Imidazol-α-Sympathomimetika, die vorzugsweise lokal zur Schleimhautabschwellung in der Nase eingesetzt werden. Zum Einsatz kommen dabei insbesondere wässrige Lösungen.

In wässriger Lösung sind Oxymetazolin und Xylometazolin instabil. In Form ihrer Hydrochlorid-Salze sind Oxymetazolin und Xylometazolin in wässriger Lösung zwar stabiler, doch kommt es auch bei diesen bei Lagerung, insbesondere bei erhöhter Temperatur, zu einem unerwünschten hydrolytischen Abbau der Wirkstoffe, insbesondere durch hydrolytische Spaltung des Imidazolrings. Es entstehen unerwünschte Abbauprodukte, die bei Verwendung der wässrigen Lösung als Arzneimittel mit der Gefahr schädliche Nebenwirkungen einhergehen können, und der Wirkstoffgehalt nimmt ab. Insgesamt ist die Haltbarkeit der wässrigen Lösung herabgesetzt.

Infolge hydrolytischer Spaltung des Imidazolinrings entstehen aus Oxymetazolin und Xylometazolin in wässriger Lösung insbesondere *N*-(2-aminoethyl)-2-[4-(1,1-dimethylethyl)-3-hydroxy-2,6-dimethylphenyl]acetamid bzw. *N*-(2-aminoethyl)-2-[4-(1,1-dimethylethyl)-2,6-dimethylphenyl]acetamid. Diese Abbauprodukte sind auch im europäischen Arzneibuch 2003 in den diese Wirkstoffe betreffenden Monografien als Verunreinigungen aufgeführt.

Der hydrolytische Abbau von Oxymetazolin und Xylometazolin kann vermieden werden, wenn anstatt des wässrigen Lösungsmittels ein nicht-wässrige Lösungsmittel eingesetzt werden, beispielsweise Öle oder organischen Lösungsmittel. Öle weisen jedoch eine vergleichsweise höhere Viskosität sowie eine schlechtere Benetzbarkeit von hydrophilen Flächen auf, was bei nasaler Anwendung einer feinen Verteilung des enthaltenen Wirkstoffes auf der Nasenschleimhaut entgegensteht. Organische Lösungsmittel sind zumeist toxikologisch nicht unbedenklich und/oder führen zu Reizungen der Nasenschleimhaut. Auch erscheinen nicht-wässrige Formulierungen auf Grund ihrer Viskosität bzw. möglicher Wechselwirkungen mit Packmitteln und Dosierungssystemen, insbesondere solchen aus Kunststoffen, weniger gut geeignet zur Entwicklung von Wirkstofflösungen. Dies gilt besonders für Sprühflaschen aus Kunststoffen, die für Rhinologika breite Anwendung finden.

Es war Aufgabe der vorliegenden Erfindung eine wässrige Lösung von Oxymetazolin und/oder Xylometazolin mit erhöhter Stabilität zur Verfügung zustellen. Insbesondere sollte der hydrolytische Abbau infolge Spaltung des Imidazolinrings vermindert sein.

Überraschenderweise wurde gefunden, dass eine stabile wässrige Lösung mit Oxymetazolin und/oder Xylometazolin erhalten werden kann, wenn diese neben dem Oxymetazolin und/oder Xylometazolin ein Zinksalz und ein Puffersalz enthält. Gegenstand der vorliegenden Erfindung ist daher eine wässrige Lösung mindestens enthaltend Oxymetazolin und/oder Xylometazolin, ein Zinksalz und ein Puffersalz.

Eine wässrige Lösung im Sinne der Erfindung liegt vor, wenn zumindest einen Teil des enthaltenen Lösungsmittels aus Wasser besteht. Als weitere Lösungsmittelbestandteile können alle Lösungsmittel enthalten sein, die für die nasalen Anwendung geeignet sind, insbesondere Alkohole wie z. B. Ethanol, Propanol, Propandiol oder Glycerol. Bevorzugt enthält die wässrige Lösung als Lösungsmittel Wasser oder Ethanol-WasserGemische, besonders bevorzugt besteht das Lösungsmittel aus Wasser.

In der erfindungsgemäßen wässrigen Lösung ist Oxymetazolin und/oder Xylometazolin vorzugsweise in Form eines seiner pharmazeutisch verträglichen Salze wie z. B. als Hydrochlorid oder als Nitrat enthalten. Besonders bevorzugt sind Oxymetazolin und/oder Xylometazolin jeweils als Hydrochlorid enthalten. Soweit in der vorliegenden Patentanmeldung Mengenangaben von Oxymetazolin bzw. Xylometazolin enthalten sind, beziehen sich diese jeweils auf die entsprechenden Hydrochlorid-Salze. Andere Salzformen von Oxymetazolin-oder Xylometazolin-Nitrat werden in einer dem jeweiligen Hydrochlorid-Salz entsprechenden äquimolaren Menge eingesetzt.

Als Zinksalz sind erfindungsgemäß alle pharmazeutisch akzeptablen Zinksalze einsetzbar. Bevorzugt sind Zinkchlorid, Zinklactat, Zinksulfat, Zinkcitrat, Zinkacetat, Zinkhistidinat, Zinkorotat, Zinkaspartat und/oder Zinkgluconat enthalten. Besonders bevorzugt ist als Zinksalz Zinkgluconat enthalten.

Puffersalze im Sinne der vorliegenden Erfindung sind die Salze schwacher Säuren mit starken Basen oder schwacher Basen mit starken Säuren, die in wässriger Lösung vollständig dissoziieren und in Gegenwart der jeweiligen, das Salz ausbildenden Säure bzw. Base ein Puffersystem ausbilden. Puffersalze, die erfindungsgemäß verwendet werden können, sind beispielsweise Alkali-Metall-Salze, insbesondere die Natrium- und/oder Kalium-Salze, von pharmazeutisch verwendbaren schwachen organischen oder anorganischen Säuren wie z. B. Essigsäure oder Citronensäure oder Borsäure. Besonders bevorzugt ist Natriumcitrat als Puffersalz enthalten.

Nach einer vorteilhaften Ausführungsform der Erfindung enthält die wässrige Lösung weiterhin eine pharmazeutisch akzeptable Säure oder Base. Als Säure oder Base können alle pharmazeutisch akzeptablen Säuren oder Basen enthalten sein, die mit den anderen Bestandteilen der erfindungsgemäßen wässrigen Lösung zu keinen Unverträglichkeiten führen. Bevorzugt ist als Säure die jeweils das Puffersalz bildende Säure, d. h. die Säureform des im Puffersalz enthaltenen Anions, oder als Base die das Puffersalz bildende Base, d. h. die Basenform des im Puffersalz enthaltenen Kations, enthalten. Beispielsweise kann in der erfindungsgemäßen wässrigen Lösung neben dem Puffersalz Natriumcitrat Citronensäure, d. h. die Säureform der im Puffersalz als Anion enthaltenen Citrat-lonen, oder NaOH, d. h. die Basenform der im Puffersalz als Kation enthaltenen Natrium-lonen, enthalten sein.

Die erfindungsgemäße wässrige Lösung kann zur nasalen Anwendung in allen zur nasalen Verabreichung geeigneten Arzneiformen, wie zum Beispiel Nasentropfen oder Nasensprays, mittels hierzu geeigneter Dosierungsvorrichtungen wie Flaschen mit Tropfeinrichtung oder Nasenspray-Pumpen appliziert werden.

Nach einer zweckmäßigen Ausführungsform der Erfindung weist die wässrige Lösung einen pH-Wert von pH 4 bis pH 7,5, vorzugsweise eine pH-Wert von pH 5,0 bis pH 7,2, besonders bevorzugt einen pH-Wert von ca. pH 6,0 auf. Die genaue Einstellung des pH-Werts kann dabei durch Zugabe der zum Puffersalz korrespondierenden Säure bzw. Base und/oder durch Zugabe einer anderen physiologisch verträglichen Säure bzw. Base erfolgen. Beispielsweise kann der gewünschte pH-Wert einer Natriumcitrat enthaltenden Lösung durch Zugabe der Säureform des im Puffersalz enthaltenen Anions, d. h. durch Zugabe von Citronensäure bzw. durch Zugabe der Basenform des im Puffersalz enthaltenen Kations, d. h. durch Zugabe von NaOH und/oder durch Zugabe einer anderen Säure bzw. Base, insbesondere durch Zugabe von Salzsäure oder Natronlauge, eingestellt werden.

Um die Verträglichkeit der wässrigen Lösung bei Anwendung auf der Nasenschleimhaut zu verbessern, ist es vorteilhaft diese als isotonische Lösung zu formulieren. Isotonizität liegt bei einer Osmolalität von ca. 280 mOsm vor. Die Einstellung der Osmolalität kann durch Variation der Mengen der neben Oxymetazolin und/oder Xylometazolin in der wässrigen Lösung enthaltenen gelösten Stoffe, d. h. des Zinksalzes, Puffersalzes sowie der gegebenenfalls weiterhin enthaltenen Stoffe und/oder durch Zugabe eines Isotonisierungsmittels, bevorzugt einem physiologisch verträgliches Salz, wie beispielsweise Natrium- oder Kaliumchlorid, oder einem physiologisch verträglichen Polyol, wie beispielsweise einem Zuckeralkohol, insbesondere Sorbitol oder Glycerin, in der zur Isotonisierung erforderlichen Konzentration erfolgen. Bevorzugt erfolgt die Einstellung der Osmolalität durch Auswahl der in der wässrigen Lösung ohnehin enthaltenden Mengen an gelösten Stoffen, sodass es nicht erforderlich ist, ein Isotonisierungsmittel zuzugeben.

Die erfindungsgemäße wässrige Lösung enthält Oxymetazolin und/oder Xylometazolin in einer Konzentration von 0,005 Gew.-% bis 1,0 Gew.%. Bevorzugt ist Oxymetazolin und/oder Xylometazolin in einer Konzentration von 0,01 Gew.-% bis 0,5 Gew.%, ganz besonders bevorzugt in einer Konzentration von zwischen 0,05 Gew.-% bis 0,1 Gew.-% enthalten.

Die erfindungsgemäße wässrige Lösung enthält dass das Zinksalz in einem Mengenanteil von 0,1 Gew.-% bis 10 Gew.-%. Bevorzugt ist das Zinksalz in einem Mengenanteil von 1,8 - 6,0 Gew.-% enthalten.

Das Puffersalz ist einem Mengenanteil von 0,01 Gew.-% bis 3,0 Gew.-% enthalten. Bevorzugt ist das Puffersalz in einem Mengenanteil von 0,2 - 1,5 Gew.-% enthalten.

Nach einer vorteilhaften Ausführungsform enthält die erfindungsgemäße wässrige Lösung 0,005 Gew.-% bis 0,1 Gew.-% Oxymetazolinhydrochlorid oder Xylometazolinhydrochlorid, 1 Gew.-% bis 10 Gew.-% Zinkgluconat und 0,5 Gew.-% bis 5 Gew.-% Citrat und weist einen pH-Wert von ca. 6 auf.

Nach einer vorteilhaften Ausführungsform der Erfindung enthält die wässrige Lösung nur einen der Wirkstoffe Oxymetazolin und Xylometazolin in Form seines Hydrochlorid-Salzes.

Die Beispiele, ohne darauf beschränkt zu sein, erläutern die Erfindung.

### Beispiel 1

| | |
|---|---|
| Oxymetazolinhydrochlorid | 2,625 g |
| Zinkgluconat | 300,00 g |
| NaOH, 1 Mol/Liter | 51,20 ml |
| Natriumcitrat | 41,440 g |
| Wasser für Injektionszwecke | 4604,735 g |
| pH-Wert | 5,99 |
| Osmolalität | 280 mOsm/l |

### Herstellungsverfahren

Wasser für Injektionszwecke wird vorgelegt. Die abgewogenen Substanzen werden unter Rühren portionsweise zugegeben und gelöst. Die fertige Lösung wird über einen 0,2 µm Sterilfilter filtriert und in die dafür vorgesehenen Behältnisse abgefüllt.

### Beispiel 2:

| | |
|---|---|
| Oxymetazolinhydrochlorid | 2,625 g |
| Zinkgluconat | 200 g |
| NaOH 1 Mol/Liter | 32 ml |
| Natriumcitrat | 65,94 g |
| Wasser für Injektionszwecke | 4699,435 g |
| pH-Wert | 6,00 |
| Osmolalität | 280 mOsm/l |

Die Herstellung der wässrigen Lösung erfolgt analog Beispiel 1.

### Beispiel 3

| | |
|---|---|
| Oxymetazolinhydrochlorid | 2,625 g |
| Zinkgluconat | 90,0 g |
| Citronensäure | 2,140 g |
| Natriumcitrat | 105,84 g |
| Wasser für Injektionszwecke | 4799,395 g |
| pH-Wert | 6,00 |
| Osmolalität | 280 mOsm/l |

### Beispiel 4 (Vergleichsbeispiel ohne Zinksalz)

| | |
|---|---|
| Oxymetazolinhydrochlorid | 2,625 g |
| Citronensäure | 8,480 g |
| Natriumcitrat | 144,100 g |
| Wasser für Injektionszwecke | 4844,795 g |
| pH-Wert | 5,99 |
| Osmolalität | 291 mOsm/l |

Die Herstellung der wässrigen Lösung erfolgt analog Beispiel 1.

### Beispiel 5

| | |
|---|---|
| Xylometazolinhydrochlorid | 1,000 g |
| Zinkgluconat | 60,00 g |
| NaOH, 1 Mol/Liter | 10,24 ml |
| Natriumcitrat | 8,288 g |
| Wasser für Injektionszwecke | 954,0 g |
| pH-Wert | 6,0 |
| Osmolalität | 290 mOsm/l |

Die Herstellung der wässrigen Lösung erfolgt analog Beispiel 1.

### Beispiel 6

| | |
|---|---|
| Oxymetazolinhydrochlorid | 0,500 g |
| Zinkgluconat | 60,00 g |
| NaOH, 1 Mol/Liter | 22,0 ml |
| Natriumcitrat | 8,288 g |
| Wasser für Injektionszwecke | 942,1 g |
| pH-Wert | 7,0 |
| Osmolalität | 302 mOsm/l |

Die Herstellung der wässrigen Lösung erfolgt analog Beispiel 1.

### Beispiel 7

| | |
|---|---|
| Oxymetazolinhydrochlorid | 0,500 g |
| Zinkgluconat | 60,00 g |
| Citronensäure | 1,441 g |
| Natriumcitrat | 8,288 g |
| Wasser für Injektionszwecke | 963,7 g |
| pH-Wert | 5,0 |
| Osmolalität | 302 mOsm/l |

Die Herstellung der wässrigen Lösung erfolgt analog Beispiel 1.

### Beispiel 8

| | |
|---|---|
| Oxymetazolinhydrochlorid | 0,525 g |
| Zinkgluconat | 60,00 g |
| Natriumacetat | 9,935 g |
| Wasser für Injektionszwecke | 959,5 g |
| pH-Wert | 6,0 |
| Osmolalität | 285 mOsm/l |

Die Herstellung der wässrigen Lösung erfolgt analog Beispiel 1.

Die Stabilität der erfindungsgemäßen Formulierung wurde in einem - Stresstest geprüft. Hierzu wurden Behältnisse enthaltend die Lösungen gemäß Beispiel 1 sowie zu Vergleichszwecken Behältnisse enthaltend Lösung gemäß Beispiel 4 bei 30°C und 65% relativer Luftfeuchtigkeit (rF), rF sowie 40°C und 75% eingelagert. Vor Einlagerung sowie nach einer Lagerzeit von 52 Wochen bzw. 26 Wochen wurden sowohl für die Bestimmung des Gehaltes an Oxymetazolin als auch für die Bestimmung von deren Zersetzungsprodukten jeweils 2 Behältnisse entnommen und mittels Hochdruckflüssigkeitschromatographie (HPLC) untersucht.

Die HPLC-Chromatographischen Untersuchungen erfolgte mit Pufferlösung pH 2,5 / Acetonitril 828/172 (V/V) als Eluenten. Säule: LiChrospher^{®} 100 CN, Detektion bei 215 nm.

Die Ergebnisse der Stabilitätsuntersuchungen sind in Tabelle 1 dargestellt.

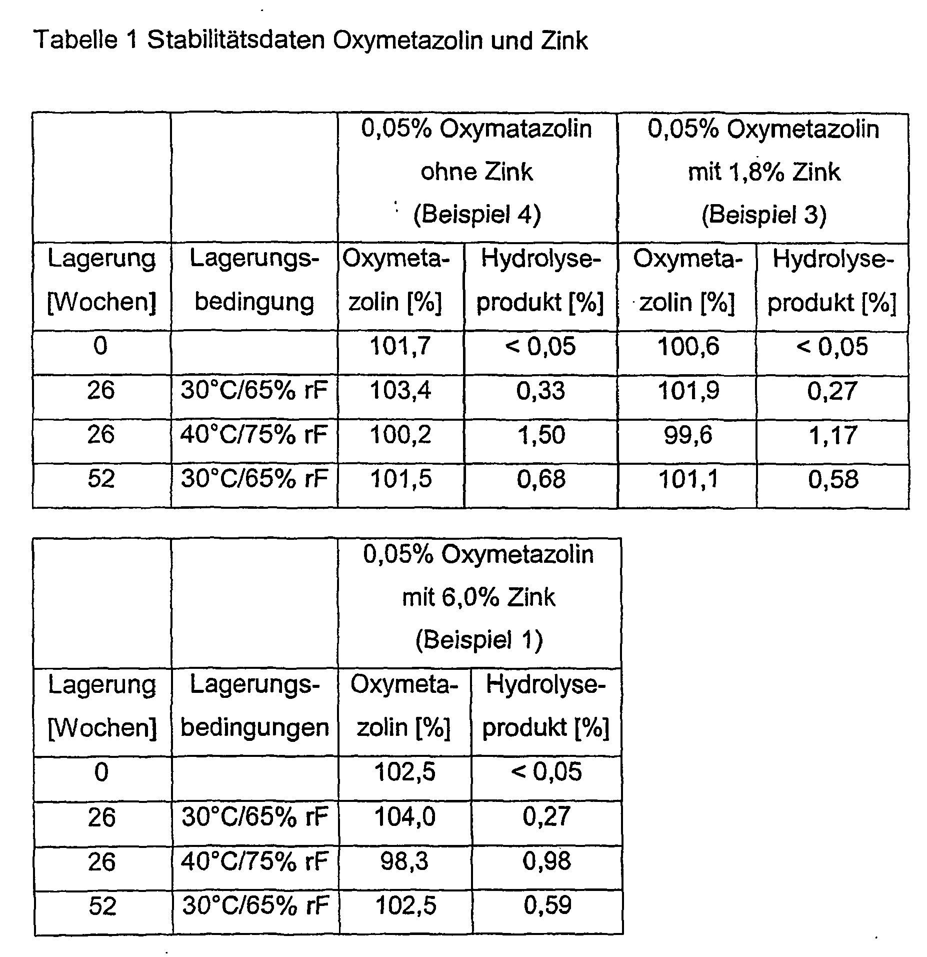

Die Ergebnisse zeigen deutlich dass die erfindungsgemäße Formulierung gegenüber der Vergleichslösung ohne Zink eine deutlich erhöhte Stabilität aufweist.

## Patentansprüche

1. Wässrige pharmazeutische Lösung mindestens enthaltend Oxymetazolin und/oder Xylometazolin, ein Zinksalz und ein Puffersalz.

2. Wässrige pharmazeutische Lösung gemäß Anspruch 1, **dadurch gekennzeichnet, dass** als Wirkstoff Oxymetazolin und/oder Xylometazolin in Form seines Hydrochlorid-Salzes enthalten ist/sind

3. Wässrige pharmazeutische Lösung gemäß Anspruch 1 oder 2, **dadurch gekennzeichnet, dass** als Zinksalz Zinkchlorid, Zinklactat, Zinksulfat, Zinkcitrat, Zinkacetat, Zinkhistidinat, Zinkorotat, Zinkaspartat und/oder Zinkgluconat, enthalten ist

4. Wässrige pharmazeutische Lösung gemäß Anspruch 3, **dadurch gekennzeichnet, dass** als Zinksalz Zinkgluconat enthalten ist

5. Wässrige pharmazeutische Lösung gemäß einem oder mehreren der Ansprüche 1 bis 4, **dadurch gekennzeichnet, dass** als Puffersalz Natriumcitrat enthalten ist

6. Wässrige Lösung gemäß einem oder mehreren der Ansprüche 1 bis 5, **dadurch gekennzeichnet, dass** weiterhin eine oder mehrere Säure/n oder eine oder mehrere Base/n enthalten ist/sind

7. Wässrige Lösung gemäß Anspruch 6, **dadurch gekennzeichnet, dass** als Säure die jeweils das Puffersalz bildende Säure, d. h. die Säureform des im Puffersalz enthaltenen Anions, oder als Base die das Puffersalz bildende Base, d. h. die Basenform des im Puffersalz enthaltenen Kations, enthalten ist

8. Wässrige pharmazeutische Lösung gemäß einem oder mehreren der Ansprüche 1 bis 7, **dadurch gekennzeichnet, dass** die Lösung ein pH-Wert von pH 4 bis pH 7,5 aufweist

9. Wässrige pharmazeutische Lösung gemäß Anspruch 8, **dadurch gekennzeichnet, dass** die Lösung ein pH-Wert von pH 5,0 bis pH 7,2, insbesondere einen pH-Wert von cirka pH 6,0, aufweist

10. Wässrige pharmazeutische Lösung gemäß einem oder mehreren der Ansprüche 1 bis 9, **dadurch gekennzeichnet, dass** die Lösung einer Osmolalität von ca. 280 mOsm aufweist

11. Wässrige pharmazeutische Lösung gemäß einem oder mehreren der Ansprüche 1 bis 10, **dadurch gekennzeichnet, dass** Oxymetazolin und/oder Xylometazolin in einer Konzentration von 0,005 Gew.-% bis 1,0 Gew.-% enthalten ist

12. wässrige pharmazeutische Lösung gemäß Anspruch 11, **dadurch gekennzeichnet, dass** die Oxymetazolin und/oder Xylometazolin in einer Konzentration von 0,01 Gew.-% bis 0,5 Gew.%, insbesondere in einer Konzentration von 0,05 Gew.-% bis 0,1 Gew.-%, enthalten ist

13. Wässrige pharmazeutische Lösung gemäß einem oder mehreren der Ansprüche 1 bis 12, **dadurch gekennzeichnet, dass** das Zinksalz in einer Konzentration von 0,1 Gew.-% bis 10 Gew.-% enthalten ist

14. Wässrige pharmazeutische Lösung gemäß einem oder mehreren der Ansprüche 1 bis 13, **dadurch gekennzeichnet, dass** das Puffersalz in einer Konzentration von 0,01 Gew.-% bis 3 Gew.-% enthalten ist

15. Wässrige pharmazeutische Lösung gemäß einem oder mehreren der Ansprüche 1 bis 14, **dadurch gekennzeichnet, dass** diese cirka 0,005 Gew.-% bis 0,1 Gew.-% Oxymetazolin und/oder Xylometazolin, 1 Gew.-% bis 10 Gew.-% Zinkgluconat, sowie einen Citratpuffer mit einem pH-Wert von cirka 6,0 enthält

16. wässrige pharmazeutische Lösung gemäß einem oder mehreren der Ansprüche 1 bis 15, **dadurch gekennzeichnet, dass** nur einer der Wirkstoffe Oxymetazolin und Xylometazolin in Form seines Hydrochlorid-Salzes enthalten ist

17. Verwendung der wässrigen pharmazeutischen Lösung gemäß einem oder mehreren der Ansprüche 1 bis 16 zur Herstellung eines Arzneimittels zur lokalen Schleimhautabschwellung in der Nase

## Claims

1. Aqueous pharmaceutical solution at least comprising oxymetazoline and/or xylometazoline, a zinc salt and a buffer salt.

2. Aqueous pharmaceutical solution according to Claim 1, **characterised in that** as active compound oxymetazoline and/or xylometazoline is/are present in the form of the hydrochloride salt thereof.

3. Aqueous pharmaceutical solution according to Claim 1 or 2, **characterised in that** as zinc salt zinc chloride, zinc lactate, zinc sulfate, zinc citrate, zinc acetate, zinc histidinate, zinc orotate, zinc aspartate and/or zinc gluconat is present.

4. Aqueous pharmaceutical solution according to Claim 3, **characterised in that** as zinc salt zinc gluconate is present

5. Aqueous pharmaceutical solution according to one or more of Claims 1 to 4, **characterised in that** as buffer salt sodium citrate is present

6. Aqueous solution according to one or more of Claims 1 to 5, **characterised in that** one or more acid(s) or one or more base(s) is/are furthermore present

7. Aqueous solution according to Claim 6, **characterised in that** as acid the respective acid forming the buffer salt, i.e. the acid form of the anion present in the buffer salt, or as base the base forming the buffer salt, i.e. the base form of the cation present in the buffer salt, is present

8. Aqueous pharmaceutical solution according to one or more of Claims 1 to 7, **characterised in that** the solution has a pH of pH 4 to pH 7.5

9. Aqueous pharmaceutical solution according to Claim 8, **characterised in that** the solution has a pH of pH 5.0 to pH 7.2, in particular a pH of about pH 6.0

10. Aqueous pharmaceutical solution according to one or more of Claims 1 to 9, **characterised in that** the solution has an osmolality of about 280 mOsm

11. Aqueous pharmaceutical solution according to one or more of Claims 1 to 10, **characterised in that** oxymetazoline and/or xylometazoline is present in a concentration of 0.005% by weight to 1.0% by weight

12. Aqueous pharmaceutical solution according to Claim 11, **characterised in that** the oxymetazoline and/or xylometazoline is present in a concentration of 0.01% by weight to 0.5% by weight, in particular in a concentration of 0.05% by weight to 0.1% by weight

13. Aqueous pharmaceutical solution according to one or more of Claims 1 to 12, **characterised in that** the zinc salt is present in a concentration of 0.1% by weight to 10% by weight

14. Aqueous pharmaceutical solution according to one or more of Claims 1 to 13, **characterised in that** the buffer salt is present in a concentration of 0.01% by weight to 3% by weight

15. Aqueous pharmaceutical solution according to one or more of Claims 1 to 14, **characterised in that** this comprises about 0.005% by weight to 0.1% by weight of oxymetazoline and/or xylometazoline, 1% by weight to 10% by weight of zinc gluconate, and a citrate buffer having a pH of about 6.0

16. Aqueous pharmaceutical solution according to one or more of Claims 1 to 15, **characterised in that** only one of the active compounds oxymetazoline and xylometazoline is present in the form of its hydrochloride salt

17. Use of the aqueous pharmaceutical solution according to one or more of Claims 1 to 16 for the preparation of a medicament for local decongestion of the mucous membrane in the nose

## Revendications

1. Solution pharmaceutique aqueuse comprenant au moins oxymétazoline et/ou xylométazoline, un sel de zinc et un sel tampon.

2. Solution pharmaceutique aqueuse selon la revendication 1, **caractérisée en ce que**, en tant que composé actif, oxymétazoline et/ou xylométazoline sous la forme de leurs sels d'hydrochlorure est/sont présent(s).

3. Solution pharmaceutique aqueuse selon la revendication 1 ou 2 , **caractérisée en ce que**, en tant que sel de zinc, du chlorure de zinc, du lactate de zinc, du sulfate de zinc, du citrate de zinc, de l'acétate de zinc, de l'histidinate de zinc, de l'orotate de zinc, de l'aspartate de zinc et/ou du gluconate de zinc est présents.

4. Solution pharmaceutique aqueuse selon la revendication 3, **caractérisée, en ce que**, en tant que sel de zinc, du gluconate de zinc est présent.

5. Solution pharmaceutique aqueuse selon une ou plusieurs des revendications 1 à 4, **caractérisée, en ce que,** en tant que sel tampon, du citrate de sodium est présent.

6. Solution pharmaceutique aqueuse selon une ou plusieurs des revendications 1 à 5, **caractérisée en ce qu'**un ou plusieurs acide(s) ou une ou plusieurs base(s) est/sont en outre présent(e)(s).

7. Solution pharmaceutique aqueuse selon la revendication 6, **caractérisée en ce que**, en tant qu'acide, l'acide respectif formant le sel tampon, c'est-à-dire la forme acide de l'anion présent dans le sel tampon, ou en tant que base, la base formant le sel tampon, c'est-à-dire la forme base du cation présent dans le sel tampon, est présent(e).

8. Solution pharmaceutique aqueuse selon une ou plusieurs des revendications 1 à 7, **caractérisée en ce que** la solution présente un pH de pH 4 à pH 7,5.

9. Solution pharmaceutique aqueuse selon la revendication 8, **caractérisée en ce que** la solution présente un pH de pH 5,0 à pH 7,2, en particulier un pH d'environ pH 6,0.

10. Solution pharmaceutique aqueuse selon une ou plusieurs des revendications 1 à 9, **caractérisée en ce que** la solution présente une osmolalité d'environ 280 mOsm.

11. Solution pharmaceutique aqueuse selon une ou plusieurs des revendications 1 à 10, **caractérisée en ce que** de l'oxymétazoline et/ou de la xylométazoline est/sont présente(s) selon une concentration de 0,005% en poids à 1,0 % en poids.

12. Solution pharmaceutique aqueuse selon la revendication 11, **caractérisée en ce que** de l'oxymétazoline et/ou de la xylométazoline est/sont présente(s) selon une concentration de 0,01 % en poids à 0,5 % en poids, en particulier selon une concentration de 0,05% en poids à 0,1 % en poids

13. Solution pharmaceutique aqueuse selon une ou plusieurs des revendications 1 à 12, **caractérisée en ce que** le sel de zinc est présent selon une concentration de 0,1 % en poids à 10% en poids.

14. Solution pharmaceutique aqueuse selon une ou plusieurs des revendications 1 à 13, **caractérisée en ce que** le sel tampon est présent selon une concentration de 0,01 % en poids à 3% en poids.

15. Solution pharmaceutique aqueuse selon une ou plusieurs des revendications 1 à 14, **caractérisée en ce qu'**elle comprend environ 0,005% en poids à 0,1% en poids d'oxymétazoline et/ou de xylométazoline, 1% à 10% en poids de gluconate de zinc et un tampon de citrate présentant un pH d'environ 6,0.

16. Solution pharmaceutique aqueuse selon une ou plusieurs des revendications 1 à 15, **caractérisée en ce que** seulement l'un des composés actifs oxymétazoline et xylométazoline est présent sous la forme de son sel d'hydrochlorure.

17. Utilisation de la solution pharmaceutique aqueuse selon une ou plusieurs des revendications 1 à 16 pour la préparation d'un médicament pour la décongestion locale de la membrane muqueuse dans le nez.
